# EUROPEAN PATENT APPLICATION

(11) **EP 4 049 705 A1**
(43) Date of publication of application: **31.08.2022**
(21) Application number: 20879041.0
(22) Date of filing: 22.10.2020
(51) Int. Cl.: A61M 25/09, A61M 25/092

(54) **WIRE FEEDING DEVICE**

(30) Priority: 23.10.2019 JP 2019192772
(71) Applicant: ASAHI INTECC CO., LTD., Seto-shi, Aichi 489-0071 (JP)
(72) Inventor: YAMAGIWA Tomoki, Tokyo 160-0023 (JP); FUJITA Yusaku, Tokyo 160-0023 (JP); NAKAMIZO Kohei, Tokyo 160-0023 (JP)
(74) Representative: Prinz & Partner mbB
(86) International application number: PCT/JP2020/039718
(87) International publication number: WO 2021/079932

(57) **Abstract**

The object is to adjust a moving amount of a wire to an appropriate amount and feed the wire with appropriate force. A wire feeding device 1 feeding a guide wire GW includes a grip portion 10 that is able to grip the guide wire GW and move in the distal end direction, a hammer 15 that is arranged on the proximal end side of the grip portion 10, and is movable in an axial direction of the guide wire GW and is able to come into contact with and separate from the grip portion 10, a push spring 16 that is able to energize the hammer 15 toward the distal end direction, a slider 20 that deforms the push spring 16 and increases energizing force on the hammer 15 toward the distal end direction, and a protruding portion 5D that releases a deformed state of the push spring 16 with the energizing force increased by the slider 20. The guide wire GW gripped by the grip portion 10 is fed toward the distal end direction by causing the hammer 15 to hit the grip portion 10 by the energizing force of the push spring 16 whose deformed state is released by the protruding portion 5D and moving the grip portion 10 toward the distal end direction.

## Description

### TECHNICAL FIELD

The invention relates to a wire feeding device that feeds a wire.

### BACKGROUND ART

To remove an occluding object that blocks a blood vessel, such as a chronic total occlusion (CTO), and improve a blood flow, there is tested whether a soft guide wire can penetrate the occluding object, for example, and if the soft guide wire does not penetrate the occluding object, the guide wire is replaced gradually with a harder antegrade guide wire.

This method requires the effort to replace the guide wires and the cost to use a plurality of guide wires.

Further, to control the guide wire to penetrate an occluding object, a technician picks and operates a guide wire by hand. Thus, the guide wire feeding distance depends on the sense of the technician.

Meanwhile, the technique described in Patent Literature 1, for example, is known as a technique capable of feeding a medical wire with a given moving amount and preferably transmitting the pressing force of the medical wire.

### CITATION LIST

### Patent Literature

Patent Literature 1: Japanese Patent Application Laid-Open No. 2016-202711

### SUMMARY OF INVENTION

### TECHNICAL PROBLEM

In the technique described in Patent Literature 1, the technician feeds a medical wire by pressing a spring. For this reason, the pressing force against the medical wire is relatively limited and may not be sufficient for the medical wire to penetrate an occluding object. Thus, the medical wire needs to be replaced to enable penetration through the occluding object. Therefore, it is not possible to solve the problems of requiring the effort to replace the medical wires and the cost to use a plurality of medical wires.

Moreover, in the technique of the Patent Literature 1, when the grip member holding a medical wire moves to the distal end side exceeding a given moving amount, the engagement between the engagement member and the grip member, which maintains the gripped state of the medical wire, is cancelled so as to prevent a moving amount of the medical wire from exceeding the given moving amount. However, the moving amount of the medical wire may vary depending on the timing when the engagement between the engagement member and the grip member is cancelled.

The inventions have been made on the basis of the above-described circumstances, and an object of the invention is to adjust a wire moving amount to an appropriate amount and feed the wire with appropriate force.

### SOLUTION TO PROBLEM

To achieve such an object, a wire feeding device according to one aspect is a wire feeding device feeding a wire in a distal end direction. The wire feeding device includes a grip portion that is able to grip the wire and move in the distal end direction, a hitting portion that is arranged on a proximal end side of the grip portion, and is movable in an axial direction of the wire and is able to come into contact with and separate from the grip portion, an elastic body that is able to energize the hitting portion toward the distal end direction, an energizing portion that deforms the elastic body and increases energizing force on the hitting portion toward the distal end direction, and a release portion that releases a deformed state of the elastic body with the energizing force increased by the energizing portion. In the wire feeding device, the wire gripped by the grip portion is fed toward the distal end direction by causing the hitting portion to hit the grip portion by the energizing force of the elastic body whose deformed state is released by the release portion and moving the grip portion toward the distal end direction.

In the above-described wire feeding device, the grip portion, the hitting portion, and the release portion may be configured to interlock with each other so that gripping the wire, moving the grip portion toward the distal end direction by a hit of the hitting portion with the grip portion, releasing the grip of the wire, and moving the grip portion toward the proximal end direction are performed in this order.

In the above-described wire feeding device, the grip portion may be movable in a given movable range in the axial direction, and may be configured to keep, when moved to a frontmost end position in the distal end direction in the movable range by a hit of the hitting portion, gripping the wire at the position.

The above-described wire feeding device may further include a release and moving portion that cancels the grip of the wire by the grip part at the frontmost end position, and moves the grip portion from the frontmost end position to a rearmost end position in the movable range while keeping a cancelled state of the grip of the wire by the grip portion.

The above-described wire feeding device may further include a power transmission mechanism that enables, with power supplied from a same power source, an operation of moving the grip portion from the frontmost end position to the rearmost end position by the release and moving portion and an operation of increasing energizing force of the elastic body by the energizing portion, as a sequence of operations.

In the above-described wire feeding device, the elastic body may be able to increase energizing force with the movement of the hitting portion toward the proximal end side, the energizing portion may include a hook engageable with a protruding portion of the hitting portion, and may be able to move the hitting portion toward the proximal end side while the hook is engaged with the protruding portion, and the release portion may release the deformed state of the elastic body by releasing the engagement between the hook of the energizing portion and the protruding portion of the hitting portion.

In the above-described wire feeding device, the grip portion may include facing grip surfaces that are capable of gripping the wire by sandwiching the wire, and the grip surface may include a surface intersecting the movable direction of the grip portion.

In the above-described wire feeding device, the grip portion may have arrangement space for arranging the wire to be gripped, and the arrangement space may be openable to an outside through an opening extending over the entire movable direction of the grip portion.

In the above-described wire feeding device, the grip portion may have arrangement space for arranging the wire to be gripped, and the arrangement space may be able to form a through hole extending in the movable direction of the grip portion.

In the above-described wire feeding device, the wire feeding device may further include a grip release portion at the time of stopping that cancels, when energization of the energizing force by the energizing portion is stopped, the grip of the wire by the grip portion while the grip portion does not move to the distal end direction.

In the above-described wire feeding device, the power source may be a power-operated motor.

In the above-described wire feeding device, the wire feeding device may further include a lever that is able to supply power to the power transmission mechanism by turning around a given axis in accordance with technician's operation.

### ADVANTAGEOUS EFFECTS OF INVENTION

In the present invention, it is possible to adjust a wire moving amount to an appropriate amount and feed the wire with appropriate force.

### BRIEF DESCRIPTION OF DRAWINGS

FIG. 1 is a perspective view of a wire feeding device according to a first embodiment.
FIG. 2 is a sectional top view of the wire feeding device in an initial state.
FIG. 3 is a sectional side view of the wire feeding device in an initial state.
FIG. 4 is a configuration diagram of a grip portion of the wire feeding device.
FIG. 5 is a perspective view of the wire feeding device in a gripping preparation state.
FIG. 6 is a sectional side view of the wire feeding device in a gripping preparation state.
FIG. 7 is a perspective view of the wire feeding device in a compressed state.
FIG. 8 is a sectional top view of the wire feeding device in a compressed state.
FIG. 9 is a sectional side view of the wire feeding device in a compressed state.
FIG. 10 is a perspective view of the wire feeding device at the start of feeding.
FIG. 11 is a sectional side view of the wire feeding device in a state immediately before the start of feeding.
FIG. 12 is a sectional side view of the wire feeding device in a state immediately after the start of feeding.
FIG. 13 is a sectional side view of the wire feeding device in a state after the completion of feeding.
FIG. 14 is a configuration diagram of a grip portion according to a modification.
FIG. 15 is a diagram for explaining a guide wire and a catheter connected to a wire feeding device according to a second embodiment, and a connector for the connection to the wire feeding device.
FIG. 16 is a diagram illustrating a connection state between a guide wire and a catheter and a connector.
FIG. 17 is a perspective view of the wire feeding device.
FIG. 18 is a perspective view of the wire feeding device to which a guide wire and a catheter are connected.
FIG. 19 is a sectional top view of the wire feeding device in an initial state.
FIG. 20 is a configuration diagram of a grip portion of the wire feeding device.
FIG. 21 is an exploded perspective view of the wire feeding device.
FIG. 22 is a sectional top view of the wire feeding device in a gripping preparation state.
FIG. 23 is a sectional top view of the wire feeding device in a state immediately before the start of feeding.
FIG. 24 is a sectional top view of the wire feeding device in a state immediately after the start of feeding.
FIG. 25 is a bottom view of the wire feeding device in a state immediately after the start of feeding.
FIG. 26 is a sectional top view of the wire feeding device in a state after the completion of feeding.

### EMBODIMENTS OF THE INVENTION

Hereinafter, the wire feeding device according to embodiments will be described with reference to the drawings. However, the present inventions are not limited to the embodiments illustrated in the drawings.

In this specification, the term "guide wire" indicates a medical guide wire that is pushed to a surgical site in a body cavity such as a blood vessel and is used to guide a catheter to the surgical site.

In this specification, the "distal end side" and the "distal end direction" indicate the side and direction, along the longitudinal direction of the guide wire (direction along the axial direction of the guide wire), where an occluding object to be penetrated by a guide wire is positioned. The "rear end side" and the "rear end direction" are opposite to the distal side and direction. Moreover, the "proximal end side" indicates a direction along the longitudinal direction of the guide wire, the direction being opposite to the distal end side. Moreover, the "distal end" indicates an end on the distal end side of an arbitrary member or region, and the "proximal end" indicates an end on the proximal end side of an arbitrary member or region.

### (First Embodiment)

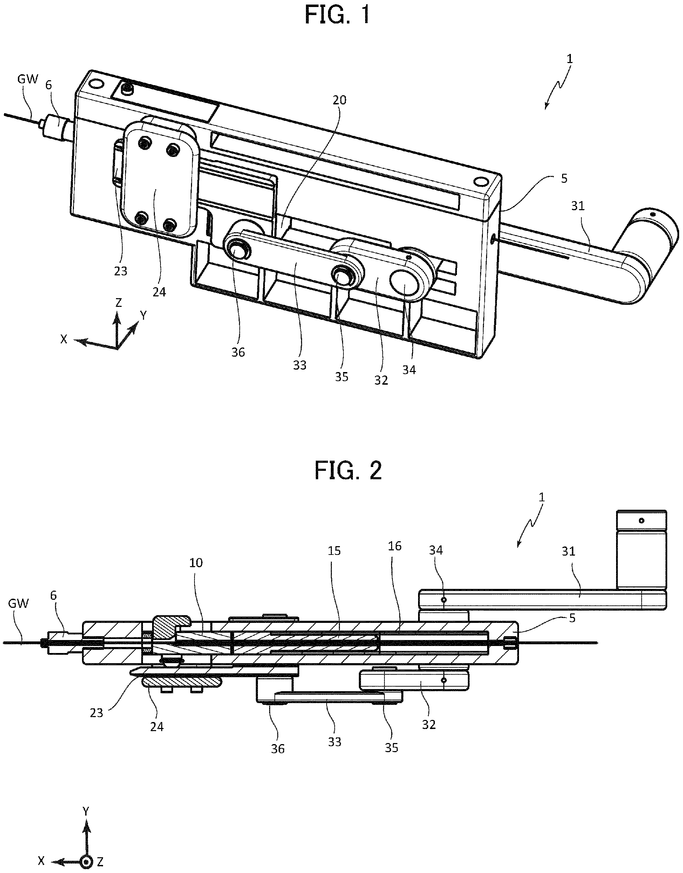

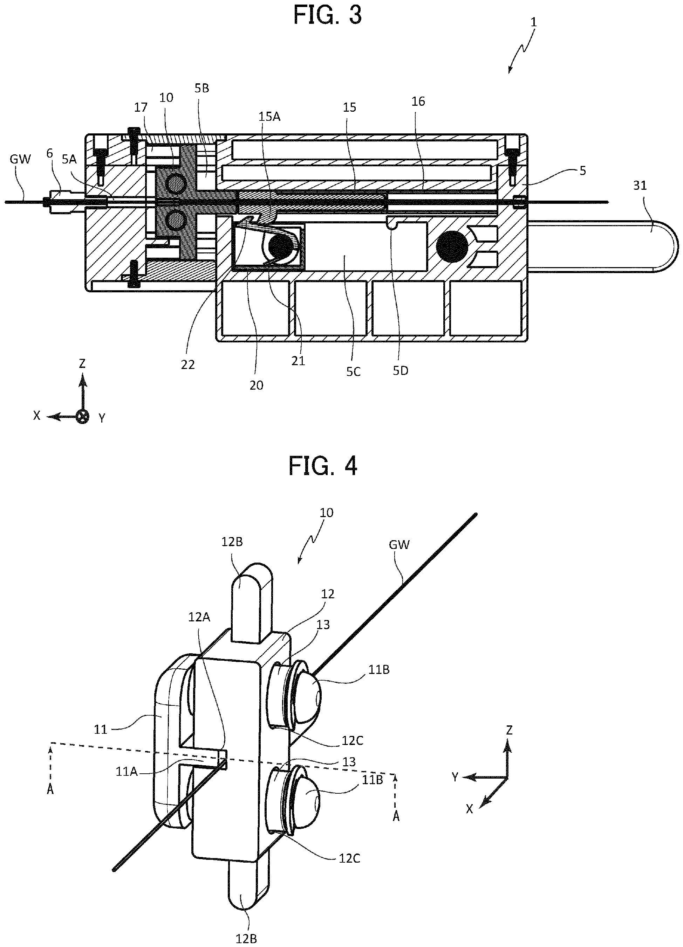

FIG. 1 is a perspective view of a wire feeding device according to the first embodiment. FIG. 2 is a sectional top view of the wire feeding device in an initial state. FIG. 3 is a sectional side view of the wire feeding device in an initial state.

A wire feeding device 1 is a device that feeds a guide wire GW as an example of a wire. The guide wire GW is pushed to a surgical site in a body cavity such as a blood vessel, for example, and is used to penetrate an occluding object in the surgical site.

The wire feeding device 1 includes a housing 5, a grip portion 10, a hammer 15, a push spring 16, a return spring 17, a slider 20, a grip release portion 23, and a holding portion 24. The hammer 15 is an example of a hitting portion. The push spring 16 is an example of an elastic body. The slider 20 is an example of an energizing portion. The slider 20 and the housing 5 are examples of a releasing portion. The wire feeding device 1 further includes a handle 31, links 32 and 33, and joints 34, 35, and 36. Here, the handle 31, the link 32, the link 33, the joints 34, 35, and 36, and the slider 20 are examples of a power transmission mechanism.

The housing 5 has a substantially rectangular parallelepiped shape with the axial direction of the guide wire GW (X-axis direction in the drawing) when the guide wire GW is attached, being the longitudinal direction. The housing 5 has a through hole 5A through which the guide wire GW is inserted. Further, the housing 5 includes a grip portion accommodating portion 5B for accommodating the grip portion 10 movably in the X-axis direction and a slider accommodating portion 5C for accommodating the slider 20 movably in the X-axis direction. Further, a protruding portion 5D is formed on the upper part on the proximal end side of the slider accommodating portion 5C. Further, on the X-axis direction side of the through hole 5A of the housing 5, an adapter 6 for connecting to an adapter on the proximal end side of a catheter is connected.

The grip portion 10 is movable in the X-axis direction in the grip portion accommodating portion 5B. The grip portion 10 is able to grip the guide wire GW.

FIG. 4 is a configuration diagram of the grip portion according to the first embodiment.

The grip portion 10 includes a first component 11, a second component 12, and grip springs 13. The first component 11 includes, for example, a protruding portion 11A extending in the X-axis direction and substantially columnar leg portions 11B extending in the Y-axis direction. The second component 12 includes a concave portion 12A extending in the X-axis direction and having a shape corresponding to the protruding portion 11A of the first component, a protruding portion 12B projecting in both directions of the Z-axis direction, and through hole portions 12C with a through hole through which the leg portion 11B can be inserted.

The first component 11 and the second component 12 are combined such that the protruding portion 11A of the first component 11 is fitted in the concave portion 12A of the second component 12 and the leg portions 11B of the first component 11 are inserted in the through holes of the through hole portion 12C of the second component 12. The grip spring 13 is attached so that energizing force is generated between the leg portion 11B of the first component 11 and the second component 12.

With such a configuration, the energizing force of the grip spring 13 acts so that the surface on the second component 12 side of the protruding portion 11A of the first component 11 and the surface on the first component 1 side of the concave portion 12A of the second component 12 approach to each other. In the present embodiment, the guide wire GW is arranged between the surface on the second component 12 side of the protruding portion 11A of the first component 11 and the surface on the first component 11 side of the concave portion 12A of the second component 12, which enables the grip portion 10 to grip the guide wire GW by the energizing force of the grip spring 13. Note that when the leg portion 11B of the first component 11 is pressed toward the first component 11 side (Y-axis direction side) and the grip spring 13 is compressed, the surface on the second component 12 side of the protruding portion 11A of the first component 11 and the surface on the first component 11 side of the concave portion 12A of the second component 12 are separated from each other, so that the grip portion 10 no longer grips the guide wire GW (grip release).

The protruding portion 12B of the second component 12 engages with a concave portion (not illustrated) extending in the X-axis direction on both wall portions in the Z-axis direction of the grip portion accommodating portion 5B of the housing 5, and acts to accurately guide the grip portion 10 in the X-axis direction.

Returning to the description using FIGS. 1 to 3, the hollow hammer 15 is arranged on the proximal end side in the X-axis direction of the grip portion 10 such that the longitudinal direction of the hammer 15 is the X-axis direction. The push spring 16 is arranged around a part on the proximal end side of the hammer 15 and on the proximal end side of the hammer 15 such that the longitudinal direction of the push spring 16 is the X-axis direction.

The hammer 15 is made of metal, for example, and is movable in the X-axis direction. The hammer 15 includes a protruding portion 15A on the side of the slider accommodating portion 5C. The protruding portion 15A can be engaged with a hook 22 described later of the slider 20. The push spring 16 is, for example, a metal spring, and is deformable (compressible) in the X-axis direction and capable of applying energizing force in the X-axis direction on the hammer 15.

The return spring 17 is, for example, a metal spring, and is deformable (compressible) in the X-axis direction and energizes the grip portion 10 toward the proximal end side. The energizing force of the return spring 17 that is applied on the grip portion 10 is smaller than the energizing force of the push spring 16 in the initial state that is applied on the grip portion 10 (a state in which the compression is not generated by the movement of the hammer 15). Thus, when the push spring 16 is in the initial state, the grip portion 10 is positioned at the frontmost end position in the movable range (the frontmost end position in the X-axis direction in the grip portion accommodating portion 5B). Meanwhile, when the push spring 16 is compressed so that no energizing force is applied on the grip portion 10, the grip portion 10 is moved to the rearmost end position in the movable range (the rearmost end position in the X-axis direction in the grip portion accommodating portion 5B) by the energizing force of the return spring 17.

In the wire feeding device 1, the handle 31, the link 32, the link 33, the slider 20, the joints 34, 35, and 36, and the grip release portion 23 form the power transmission mechanism.

The handle 31 is a handle for a technician using the wire feeding device 1 to manually perform a rotation operation. One end of the handle 31 and one end of the link 32 are connected through the joint 34 such that the rotational force is transmittable from the handle 31 to the link 32. In the present embodiment when the handle 31 is rotated once around the joint 34, the link 32 is rotated once.

The other end of the link 32 and one end of the link 33 are rotatably connected through the joint 35. The length of the link 33 is longer than that of the link 32. The other end of the link 33 and, the slider 20, and the grip release portion 23 are rotatably connected through the joint 36.

In this power transmission mechanism, when the handle 31 is rotated, the link 32 is rotated. With the rotation of the link 32, the link 33 moves with the movement of the slider 20 and the grip release portion 23 in the X-axis direction.

The slider 20 includes the hook 22 that engages with the protruding portion 15A of the hammer 15 and a torsion spring 21 that energizes the hook 22 in the Z-axis direction.

When the slider 20 moves from the frontmost end position toward the proximal end side in the X-axis direction within the moving range of the slider 20, the hook 22 engages with the protruding portion 15A of the hammer 15. When the slider 20 further moves, the hammer 15 is moved to the proximal end side, so that the push spring 16 is compressed. When the slider 20 approaches the rearmost end position, the hook 22 (torsion spring 21) is pushed downward by the protruding portion 5D of the housing 5, thereby releasing the engagement between the hook 22 and the protruding portion 15A of the hammer 15. As a result, the deformed state (compressed state) of the push spring 16 is released, so that the push spring 16 pushes the hammer 15 in the X-axis direction.

The grip release portion 23 is a plate-shaped member in which the surface on the housing 5 side on the distal end side in the X-axis direction has an inclination smaller in thickness toward the distal end side. When the grip release portion 23 is moved to the distal end side in the X-axis direction, it enters between the holding portion 24 and the leg portion 11B of the grip portion 10, compresses the grip spring 13, and moves the leg portion 11B of the grip portion 10 toward the Y-axis positive direction. Thus, the surface on the second component 12 side of the protruding portion 11A of the first component 11 and the surface on the first component 11 side of the concave portion 12A of the second component 12 are separated from each other, thereby releasing the grip of the guide wire GW.

The following will specifically describe a use method of the wire feeding device 1 according to the embodiment and the operation of the wire feeding device 1 when used, with reference to the drawings. In the wire feeding device 1, the grip portion 10, the hammer 15, and the slider 20 are configured to interlock with each other so that gripping the guide wire GW, moving the grip portion 10 toward the distal end direction of the guide wire GW, releasing the grip of the guide wire GW, and moving the grip portion 10 toward the rear end direction are performed in this order. Note that it is assumed that the wire feeding device 1 is initially in a state (initial state) not gripping the guide wire GW, as illustrated in FIG. 1.

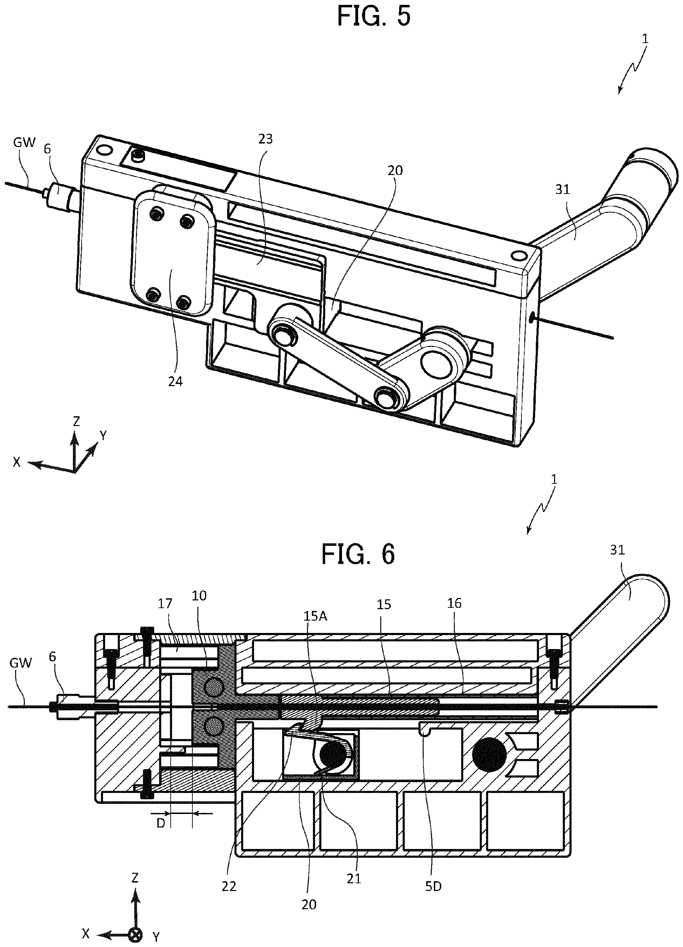

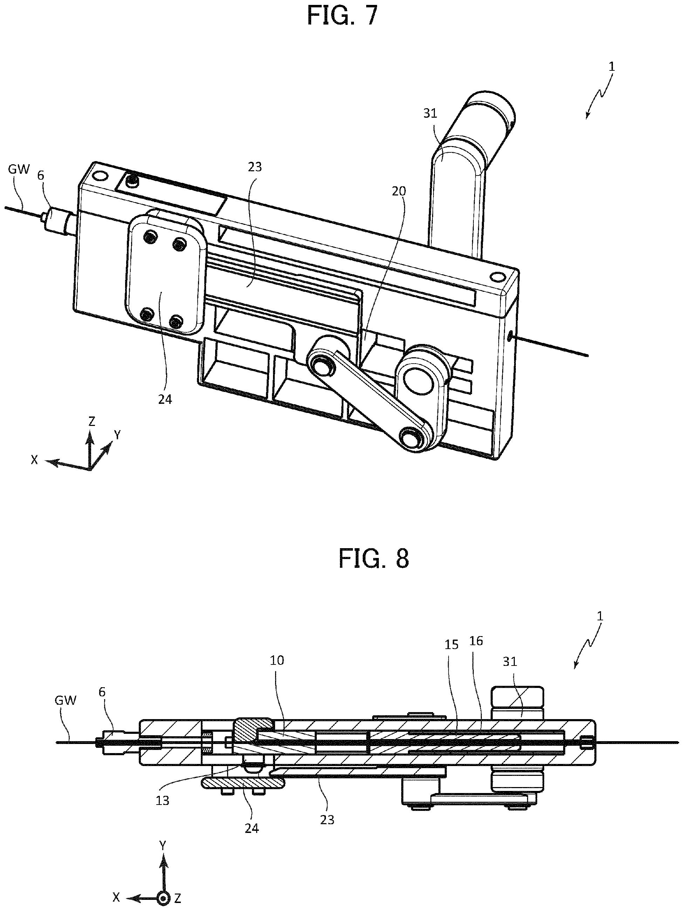

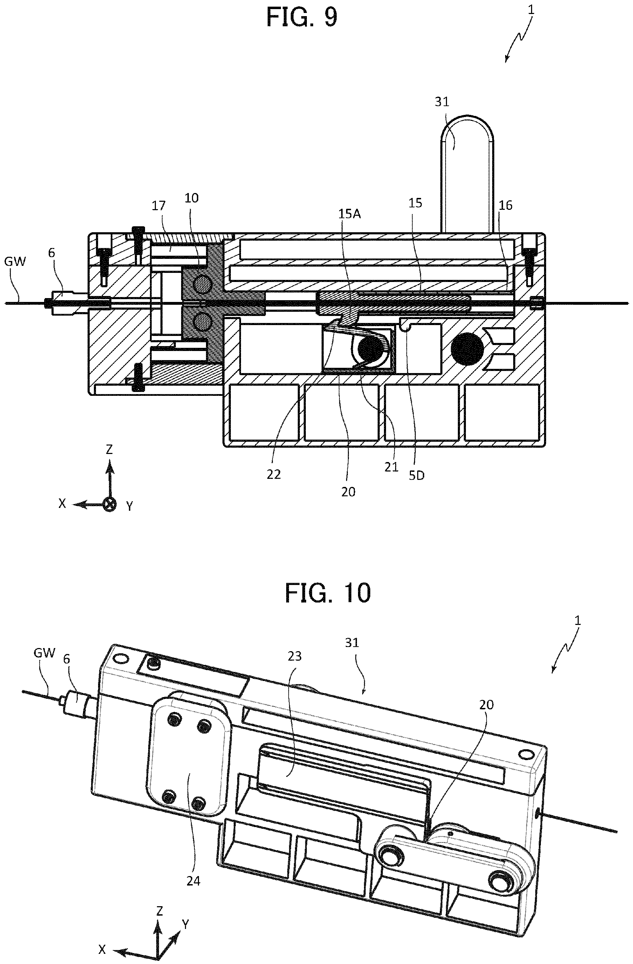

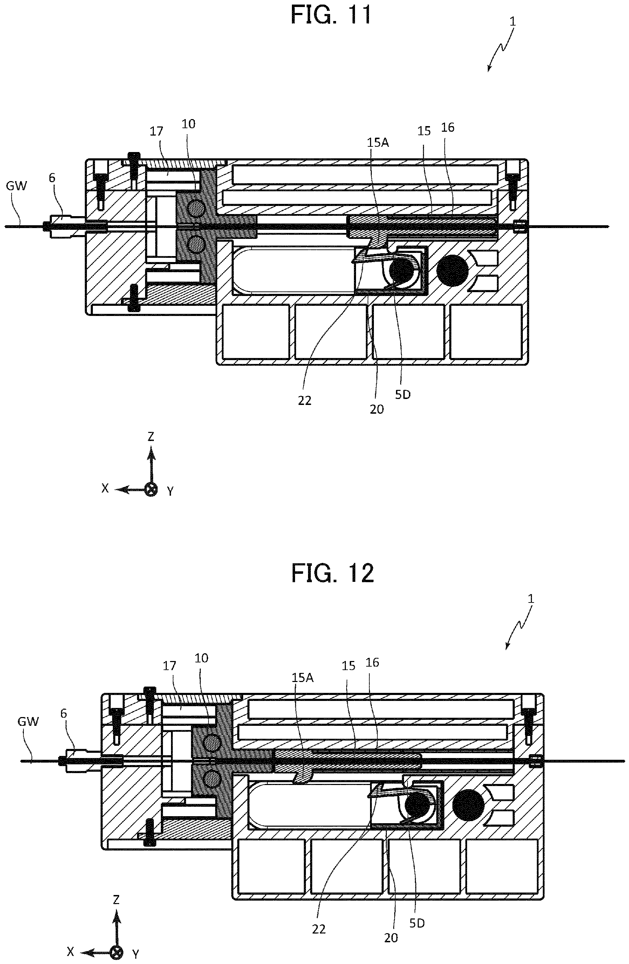

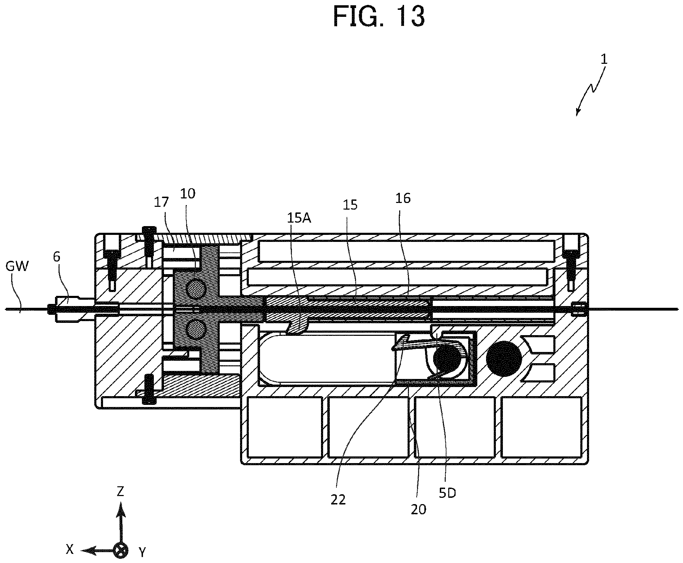

Here, FIG. 5 is a perspective view of the wire feeding device in a gripping preparation state, and FIG. 6 is a sectional side view illustrating a gripping preparation state. FIG. 7 is a perspective view of the wire feeding device in a compressed state. FIG. 8 is a sectional top view illustrating a compressed state. FIG. 9 is a sectional side view in a compressed state. FIG. 10 is a perspective view illustrating a feeding start state of the wire feeding device. FIG. 11 is a sectional side view illustrating a state immediately before the start of feeding. FIG. 12 is a sectional side view illustrating a state immediately after the start of feeding. FIG. 13 is a sectional side view of the wire feeding device in a state after the completion of feeding.

First, the guide wire GW is inserted into a blood vessel, and then the guide wire GW is pushed to an obstructed site along the blood vessel. Next, after the distal end of the guide wire GW reaches the obstructed site, a catheter (not illustrated) is pushed to the obstructed site using the guide wire GW as a guide. Then, the proximal end side of the guide wire GW is inserted into the through hole 5A of the housing 5, and the adapter on the proximal end side of the catheter is connected to the adapter 6 of the wire feeding device 1.

In this case, as illustrated in FIG. 2, the grip release portion 23 compresses the grip spring 13, and the surface on the second component 12 side of the protruding portion 11A of the first component 11 and the surface on the first component 11 side of the concave portion 12A of the second component 12 are separated from each other, and the grip of the guide wire GW by the grip portion 10 is released.

Next, when the handle 31 is rotated from the state illustrated in FIG. 1 to the state illustrated in FIG. 5, the slider 20 and the grip release portion 23 slide toward the proximal end side. Because the protruding portion 15A of the hammer 15 is engaged with the hook 22 of the slider 20, as illustrated in FIG. 6, the hammer 15 is moved toward the proximal end side with the movement of the slider 20, so that the push spring 16 is compressed.

Here, the grip portion 10 is no longer pushed by the hammer 15. Thus, as illustrated in FIG. 6, the grip portion 10 slides to the rearmost end in the movable range by the energizing force of the return spring 17. In this manner, the grip portion 10 moves by a distance D from the frontmost end (initial position) to the rearmost end in the movable range. The distance D corresponds to a feeding amount per one time by the wire feeding device 1. For example, when the feeding amount per one time by the wire feeding device 1 is 2 mm, the grip portion 10 slides to the rear end side by 2 mm from the initial position. At this time, the grip release portion 23 is configured to be at a position where the grip spring 13 of the grip portion 10 is compressed, which keeps the state where the grip portion 10 does not grip the guide wire GW.

Further, when the handle 31 is rotated from the state illustrated in FIG. 5 to the state illustrated in FIG. 7, the slider 20 and the grip release portion 23 further slide toward the proximal end side. Because the hook 22 of the slider 20 remains engaged with the protruding portion 15A of the hammer 15, as illustrated in FIG. 9, the hammer 15 is moved toward the proximal end side with the movement of the slider 20, so that the push spring 16 is further compressed.

Here, the grip release portion 23 deviates from the position where the grip spring 13 of the grip portion 10 is compressed, as illustrated in FIG. 8. Therefore, the guide wire GW between the surface on the second component 12 side of the protruding portion 11A of the first component 11 and the surface on the first component 11 side of the concave portion 12A of the second component 12 is gripped.

Further, when the handle 31 is rotated from the state illustrated in FIG. 7 to the state illustrated in FIG. 10, the slider 20 and the grip release portion 23 further slide toward the proximal end side. As illustrated in FIG. 11, the hook 22 (torsion spring 21) of the slider 20 is pushed down by the protruding portion 5D of the housing 5, whereby the engaging state of the hook 22 with the protruding portion 15A of the hammer 15 is released.

In this manner, as illustrated in FIG. 12, the energizing force of the push spring 16 is applied on the movement of the hammer 15 toward the distal end direction, so that the hammer 15 moves toward the distal end direction, and the distal end side of the hammer 15 hits the proximal end side of the grip portion 10.

As a result, as illustrated in FIG. 13, the grip portion 10 gripping the guide wire GW moves toward the distal end direction by the impact of a hit with the hammer 15, and stops at the frontmost end position of the grip portion 10. Here, the grip portion 10 is not in contact with the grip release portion 23, thereby keeping the state of gripping the guide wire GW.

Therefore, the grip portion 10 moves from the rearmost end position to the frontmost end position while keeping the state of gripping the guide wire GW. As a result, the guide wire GW is fed to the distal end side by the distance D from the rearmost end position to the frontmost end position of the grip portion 10.

Thereafter, when the handle 31 is rotated from the state illustrated in FIG. 10, the slider 20 and the grip release portion 23 slide toward the distal end side. When the rotation amount of the handle 31 reaches one round from the start, the initial states as illustrated in FIG. 1 is restored. Note that if it is necessary to further feed the guide wire GW, the handle 31 may be further rotated by a necessary circumference.

As described above, the wire feeding device 1 according to the present embodiment is able to feed the guide wire GW by only an appropriate amount by applying impact force due to the energizing force accumulated in the push spring 16 on the guide wire GW. In this manner, it is possible to apply impact force on the guide wire GW, which allows the guide wire GW to penetrate an occluding object more effectively.

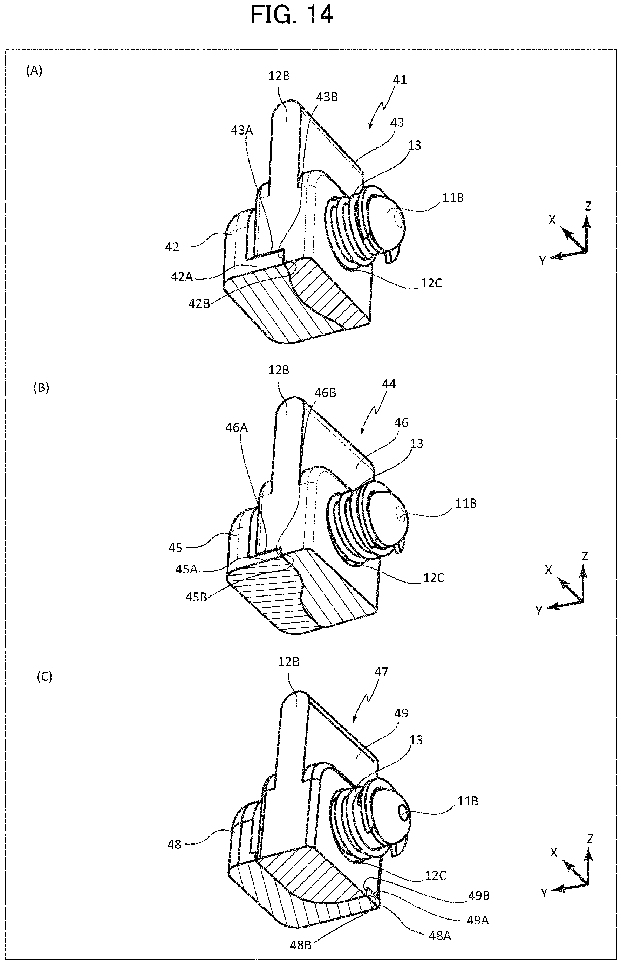

Next, a grip portion according to a modification will be described. FIG. 14 is a configuration diagram of the grip portion according to a modification. FIG. 14 is a perspective view illustrating a state in which each grip portion according to a modification is cut on the X-Y plane along the line A-A in FIG. 4. The similar parts as those of the grip portion illustrated in FIG. 4 are designated by the same reference signs.

In the grip portion 10 according to the first embodiment, the moving direction (X direction) of the grip portion 10 matches the direction of the guide wire GW sandwiched by the grip portion 10. That is, the moving direction of the grip portion 10 matches the direction in which the guide wire GW is likely to slide against the grip portion 10. Therefore, depending on the moving force of the grip portion 10 and the resistance force to the movement of the guide wire GW in a moving direction (for example, the resistance force caused by the hardness of an obstructed site in contact with the distal end of the guide wire GW), the guide wire GW may slide. Each modification is configured to prevent the guide wire GW from sliding in this manner.

A grip portion 41 illustrated in FIG. 14(A) includes a first component 42 in place of the first component 11 of the grip portion 10, and a second component 43 in place of the second component 12 of the grip portion 10.

The first component 42 includes a protruding portion 42A extending in the X-axis direction and the substantially columnar leg portion 11B extending in the Y-axis direction. The protruding portion 42A is formed such that the width in the Y-axis direction is short (low in height) in the middle part in the X-axis direction of the grip portion 41. Therefore, a surface (grip surface) 42B of the protruding portion 42A that faces the second component 43 and sandwiches the guide wire GW together with the second component 43 is formed to be concave in the Y-axis direction, and has a surface intersecting the movable direction (X-axis direction) of the grip portion 41. In the present embodiment, the end portion on the X-axis positive direction side (a direction of the arrow in the drawing) of the protruding portion 42A and the end portion on the X-axis negative direction side thereof are positioned on a straight line parallel to the X axis.

Further, the second component 43 includes a concave portion 43A having a shape corresponding to the protruding portion 42A of the first component 42, the protruding portion 12B, and the through hole portion 12C.

A surface (grip surface) 43B of the concave portion 43A that sandwiches the guide wire GW together with the first component 42 is formed to protrude in the Y-axis positive direction. In the present embodiment, the end portion on the X-axis positive direction side (a direction of the arrow in the drawing) of the concave portion 43A and the end portion on the X-axis negative direction side thereof are positioned on a straight line parallel to the X axis.

With such a configuration, the moving direction (X-axis direction) of the grip portion 41 and the direction of the guide wire GW gripped by the grip portion 41 do not completely match. That is, there exists a part where the direction of the guide wire GW is different from the moving direction of the grip portion 41. Thus, force of a component causing the grip portion 41 to directly push the guide wire GW is generated between the grip portion 41 and the guide wire GW. Therefore, it is possible to appropriately prevent the guide wire GW from sliding when the grip portion 41 grips and moves the guide wire GW. Further, in this example, the end portion on the X-axis positive direction side (a direction of the arrow in the drawing) of the protruding portion 42A and the end portion on the X-axis negative direction side thereof are positioned on a straight line parallel to the X axis, and the end portion on the X-axis positive direction side (a direction of the arrow in the drawing) of the concave portion 43A and the end portion on the X-axis negative direction side thereof are positioned on a straight line parallel to the X axis. Thus, the guide wire GW can be linearly arranged on the distal end side and the proximal end side of the grip portion 41. This makes it easy to handle the guide wire GW in the wire feeding device 1.

A grip portion 44 illustrated in FIG. 14(B) includes a first component 45 in place of the first component 11 of the grip portion 10 and a second component 46 in place of the second component 12 of the grip portion 10.

The first component 45 includes a protruding portion 45A extending in the X-axis direction and the leg portion 11B. The protruding portion 45A is formed such that the width in the Y-axis direction is shorter toward the X-axis negative direction and specifically, the protruding portion 45A has a substantially S-shape on the X-Y plane. Therefore, a surface (grip surface) 45B of the protruding portion 45A that faces the second component 46 and sandwiches the guide wire GW together with the second component 46, has a surface intersecting the movable direction (X-axis direction) of the grip portion 44.

Further, the second component 46 includes a concave portion 46A having a shape corresponding to the protruding portion 45A of the first component 45, the protruding portion 12B, and the through hole portion 12C.

A surface (grip surface) 46B of the concave portion 46A that grips the guide wire GW together with the first component 45 is formed to be concave and to protrude in the Y-axis direction.

With such a configuration, the moving direction (X-axis direction) of the grip portion 44 and the direction of the guide wire GW gripped by the grip portion 44 do not completely match. That is, there exists a part where the direction of the guide wire GW is different from the moving direction of the grip portion 44. As a result, force of a component causing the grip portion 44 to directly push the guide wire GW is generated between the grip portion 44 and the guide wire GW. Therefore, it is possible to appropriately prevent the guide wire GW from sliding when the grip portion 44 grips and moves the guide wire GW.

A grip portion 47 illustrated in FIG. 14(C) includes a first component 48 in place of the first component 11 of the grip portion 10 and a second component 49 in place of the second component 12 of the grip portion 10.

The first component 48 includes a protruding portion 48A extending in the X-axis direction and the leg portion 11B. The protruding portion 48A is formed to be curved such that the width in the Y-axis direction is longer toward the X-axis negative direction. Therefore, a surface (grip surface) 48B of the protruding portion 48A that faces the second component 49 and sandwiches the guide wire GW together with the second component 49, has a surface intersecting the movable direction (X-axis direction) of the grip portion 47.

Further, the second component 49 includes a concave portion 49A having a shape corresponding to the protruding portion 48A of the first component 48, the protruding portion 12B, and the through hole portion 12C.

A surface (grip surface) 49B of the concave portion 49A that grips the guide wire GW together with the first component 48 is formed to be curved in the Y-axis negative direction toward the X-axis negative direction.

With such a configuration, the moving direction (X-axis direction) of the grip portion 47 and the direction of the guide wire GW gripped by the grip portion 47 do not completely match. That is, there exists a part where the direction of the guide wire GW is different from the moving direction of the grip portion 47. As a result, force of a component causing the grip portion 47 to directly push the guide wire GW is generated between the grip portion 47 and the guide wire GW. Therefore, it is possible to appropriately prevent the guide wire GW from sliding when the grip portion 47 grips and moves the guide wire GW.

### (Second Embodiment)

A wire feeding device 51 according to the second embodiment is a device that feeds a guide wire GW as an example of the wire. The guide wire GW is pushed to a surgical site in a body cavity such as a blood vessel, for example, and is used to penetrate an obstructing object in the surgical site, for example. The wire feeding device 51 is used by connecting a catheter through which the guide wire GW is inserted to the device.

Before describing the details of the wire feeding device 51, a guide wire and a catheter connected to the wire feeding device 51 will be described.

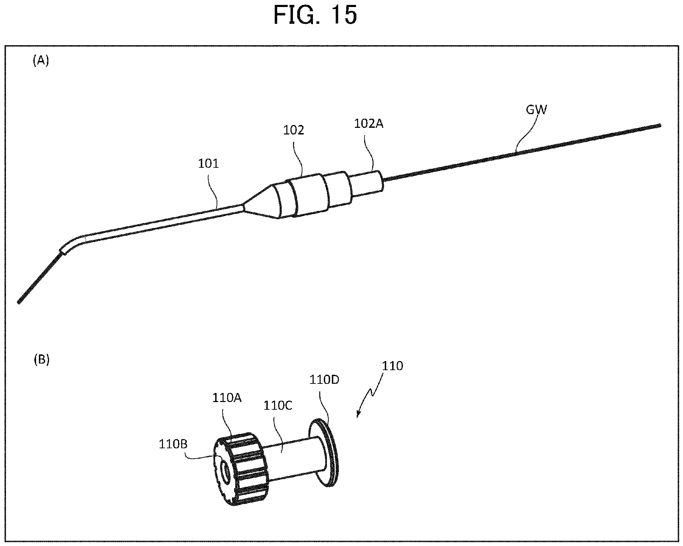

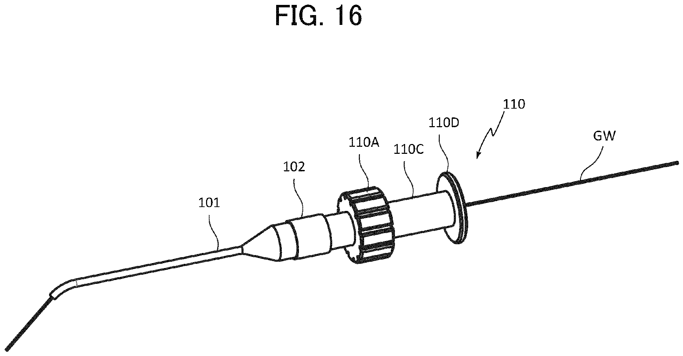

FIG. 15 is a diagram for explaining a guide wire and a catheter connected to a wire feeding device according to the second embodiment and a connector for the connection to the wire feeding device. FIG. 16 is a diagram illustrating a connection state between the guide wire and the catheter and the connector.

The guide wire GW is inserted through a hollow catheter 101. A catheter hub 102 for adjusting the direction of the catheter 101 is non-rotatably attached to one end side of the catheter 101. In the example of FIG. 15(A), the left side of the drawing is the inside of the patient's body (distal end side), and the right side of the drawing is the outside of the patient's body (proximal end side).

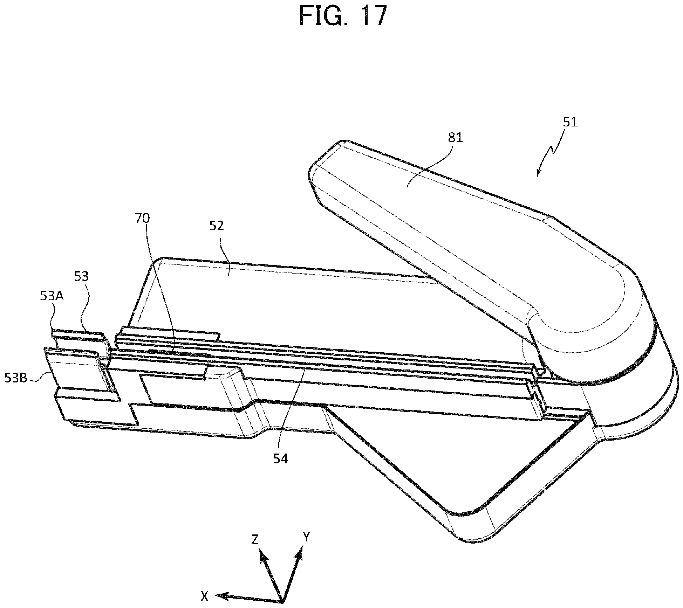

The catheter 101 is connected to a connector 110 as illustrated in FIG. 16, and is connected to the wire feeding device 51 through the connector 110. As illustrated in FIG. 15(B), the connector 110 includes a dial portion 110A, an attachment portion 110C, and a rear end portion 110D. The dial portion 110A is a portion for a technician to operate the direction of the catheter 101 connected to the connector 110. The attachment portion 110C is a portion formed in a cylindrical shape for the attachment to a connector connection portion 53 (see FIG. 17) described later of the wire feeding device 51. The length in the axial direction of the attachment portion 110C is substantially the same as the width in the X-axis direction of connecting pieces 53A and 53B described later of the connector connection portion 53. The rear end portion 110D is formed in a disk shape having a diameter larger than that of the cylinder of the attachment portion 110C. The rear end portion 110D acts to position the connector 110 in the X-axis direction with respect to the connector connection portion 53.

The connector 110 has a through hole 110B extending in the longitudinal direction. The through hole 110B is configured to engage with a rear end portion 102A of the catheter hub 102. When the rear end portion 102A of the catheter hub 102 is engaged with the through hole 110B of the connector 110, the catheter hub 102 and the connector 110 can be integrally rotated.

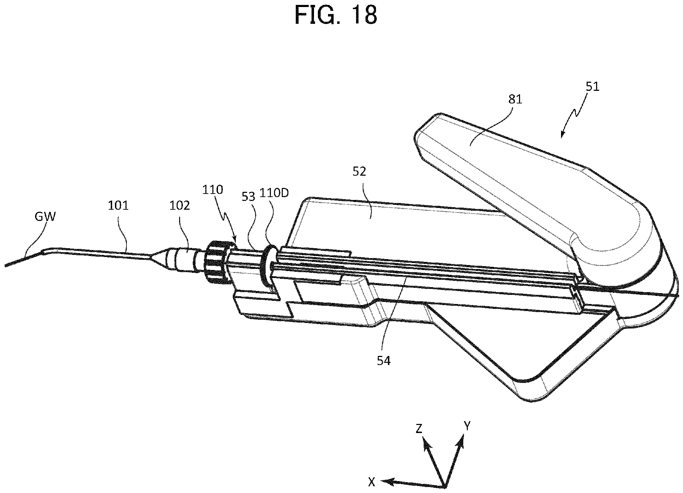

FIG. 17 is a perspective view of the wire feeding device, and FIG. 18 is a perspective view of the wire feeding device to which a guide wire and a catheter are connected.

The wire feeding device 51 includes a housing 52, a lever 81, the connector connection portion 53, a guide wire accommodating portion 54, and a grip portion 70.

The housing 52 has a substantially rectangular parallelepiped shape with the axial direction of the guide wire GW (X-axial direction in the drawing) being the longitudinal direction when the guide wire GW is attached. The housing 52 includes therein various components described later for gripping and feeding the guide wire GW in addition to the grip portion 70. The lever 81 is turnable with a lever rotation shaft 82 described later as a center, and is a portion operated by a technician to feed the guide wire GW. In the present embodiment, the technician can feed the guide wire GW by gripping the lever 81 and the housing 52 with one hand.

The connector connection portion 53 is a portion for connecting the attachment portion 110C of the connector 110, and includes a pair of connecting pieces 53A and 53B extending in the X-axis direction. The connection pieces 53A and 53B are formed of, for example, elastic bodies such as resin, and sandwich the outer peripheral surface of the attachment portion 110C of the connector 110 from both sides in the Y-axis direction to rotatably connect the connector 110. The guide wire accommodating portion 54 is a portion for accommodating the guide wire GW to be fed, and is formed in a concave shape extending in the X-axis direction and being open in the Z-axis positive direction over the entire X-axis direction. The grip portion 70 is a portion that can grip the guide wire GW and can move the guide wire GW in the X-axis direction, and is arranged in the middle part in the X-axis direction of the guide wire accommodating portion 54 such that grip surface portions 71A and 72A described later are open to the outside. In the present embodiment, when the guide wire GW is placed on the surface in the Z-axis negative direction of the guide wire accommodating portion 54 (also referred to here as a bottom surface), the guide wire GW is arranged in space (arrangement space) between the grip surfaces holding the guide wire GW that are the surface portion 71A and the gripping surface portion 72A of the grip portion 70.

To connect the catheter 101 and the guide wire GW to the wire feeding device 51, the guide wire GW at a part on the more proximal end side than the connector 110 is placed on the bottom surface of the concave portion of the guide wire accommodating portion 54, and then the attachment portion 110C of the connector 110 connected to the catheter 101 of the guide wire GW is fitted between the connection pieces 53A and 53B of the connector connection portion 53 for attachment. FIG. 18 illustrates the state in which the catheter 101 and the guide wire GW are connected to the wire feeding device 51 in this manner. In the state where the catheter 101 and the guide wire GW are connected to the wire feeding device 51 in this manner, the direction of the catheter 101 can be easily adjusted by the technician rotating the operation dial 110A of the connector 110. Further, in this state, a gap is secured between the surface on the X-axis negative direction side (a direction opposite to the direction of the arrow on the X axis) of the rear end portion 110D of the connector 110 and the surface on the X-axis positive direction side of the housing 52. Thus, liquid such as blood and chemicals that have passed through the catheter 101 easily flow down from the gap, which prevents the components of the housing 52 from coming into contact with the liquid. Further, when the lever 81 is not operated, the guide wire GW is not gripped as described later. Thus, it is possible to adjust the direction of the guide wire GW by turning the guide wire GW.

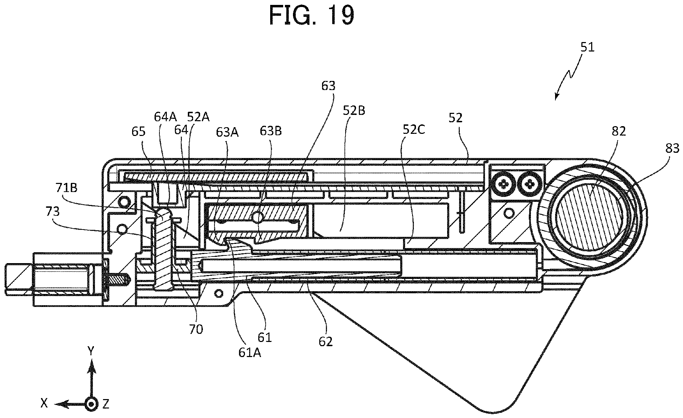

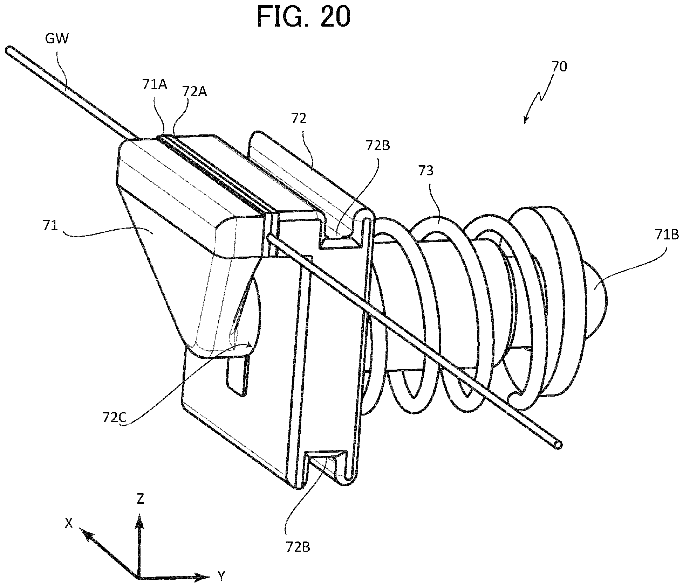

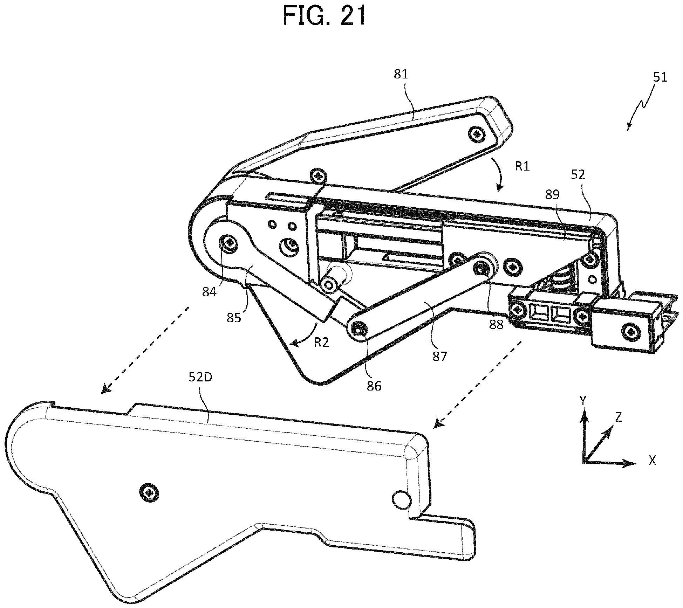

Next, the wire feeding device 51 will be described in detail. FIG. 19 is a sectional top view of the wire feeding device in an initial state. FIG. 20 is a configuration diagram of a grip portion of the wire feeding device. FIG. 21 is an exploded perspective view of the wire feeding device. FIG. 21 illustrates a state in which a partial housing 52D on the Z-axis negative direction side is removed from the wire feeding device 51.

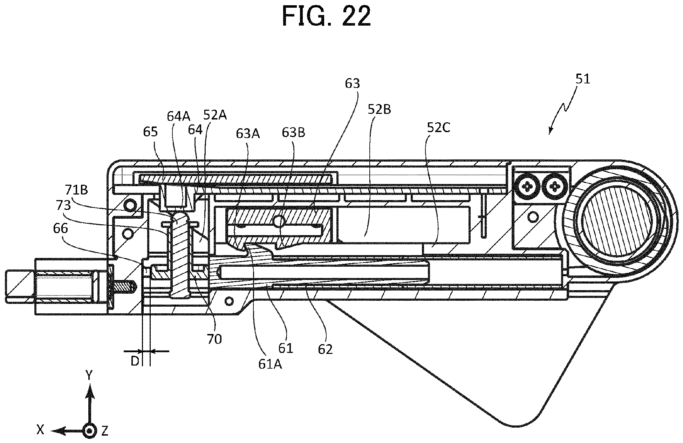

As illustrated in FIG. 19, the wire feeding device 51 includes the housing 52, the grip portion 70, a hammer 61, a push spring 62, a return spring 66 (see FIG. 22), a slider 63, a grip release drive portion 64, and a grip release portion 65. The hammer 61 is an example of a hitting portion. The push spring 62 is an example of an elastic body. The slider 63 is an example of an energizing portion. The slider 63 and the housing 52 are examples of a releasing portion. The grip release drive portion 64 and the grip release portion 65 are examples of a grip release portion at the time of stopping. As illustrated in FIG. 21, the wire feeding device 51 further includes the lever 81, links 85 and 87, and joints 84, 86, and 88, and a slider 89. Here, the lever 81, the links 85 and 87, the joints 84, 86, and 88, and the slider 89 are examples of a power transmission mechanism.

The housing 52 has a substantially rectangular parallelepiped shape with the axial direction of the guide wire GW (X-axial direction in the drawing) being longitudinal when the guide wire GW attached. Further, the housing 52 includes a grip portion accommodating portion 52A for accommodating the grip portion 70 movably in the X-axis direction and a slider accommodating portion 52B for accommodating the slider 63 movably in the X-axis direction. Further, a protruding portion 52C is formed on the upper part on the proximal end side of the slider accommodating portion 52B.

The grip portion 70 is movable in the X-axis direction in the grip portion accommodating portion 52A. The grip portion 70 is able to grip the guide wire GW.

As illustrated in FIG. 20, the grip portion 70 includes a first component 71, a second component 72, and a grip spring 73. The first component 71 includes, for example, the grip surface portion 71A extending in the X-axis direction and a substantially columnar leg portion 71B extending in the Y-axis direction. The second component 72 is formed to extend in the X-axis direction and includes the grip surface portion 72A having a surface facing the grip surface portion 71A of the first component 71, a concave portion 72B recessed in both directions in the Z-axis direction, and a through hole portion 72C with a through hole through which the leg portion 71B can be inserted.

The first component 71 and the second component 72 are combined such that the leg portion 71B of the first component 71 is inserted in the through hole of the through hole portion 72C of the second component 72. The grip spring 73 is attached so that energizing force is generated between the leg portion 71B of the first component 71 and the second component 72.

With such a configuration, the energizing force of the grip spring 73 acts so that the surface (grip surface) on the second component 72 side of the grip surface portion 71A of the first component 71 and the surface (grip surface) on the first component 71 side of the grip surface portion 72A of the second component 72 approach to each other. In the present embodiment, space (arrangement space) is secured between the grip surface on the second component 72 side of the grip surface portion 71A of the first component 71 and the grip surface on the first component 71 side of the grip surface portion 72A of the second component 72, whereby the guide wire GW can be arranged in the space, and the grip portion 70 is able to grip the guide wire GW by the energizing force of the grip spring 73. This arrangement space can be opened to the outside by an opening extending over the entire movable direction of the grip portion 70. In this embodiment, the guide wire GW is placed on the concave portion of the guide wire accommodating portion 54, whereby it is possible to position the guide wire GW substantially in the middle in the Z-axis direction of the grip surface of the grip surface portion 71A of the first component 71 and the grip surface of the grip surface portion 72A of the second component 72. Meanwhile, when the leg portion 71B of the first component 71 is pressed toward the first component 71 side (Y-axis negative direction side) and the grip spring 73 is compressed, the grip surface of the grip surface portion 71A of the first component 71 and the grip surface of the grip surface portion 72A of the second component 72 are separated from each other, and space is generated therebetween. As a result, the grip portion 70 no longer grips the guide wire GW (grip release).

The concave portion 72B of the second component 72 engages with a protruding portion (not illustrated) extending in the X-axis direction on both wall portions in the Z-axis direction of the grip portion accommodating portion 52A of the housing 52, and acts to accurately guide the grip portion 70 in the X-axis direction.

As illustrated in FIG. 19, the hollow hammer 61 is arranged on the proximal end side in the X-axis direction of the grip portion 70 such that the longitudinal direction of the hammer 61 is the X-axis direction. The push spring 62 is arranged around a part on the proximal end side of the hammer 61 and on the proximal end side of the hammer 61 such that the longitudinal direction of the push spring 62 is the X-axis direction.

The hammer 61 is made of metal, for example, and is movable in the X-axis direction. The hammer 61 includes a protruding portion 61A on the side of the slider accommodating portion 52B. The protruding portion 61A can be engaged with a hook 63A described later of the slider 63. The push spring 62 is, for example, a metal spring, and is deformable (compressible) in the X-axis direction and capable of applying energizing force in the X-axis direction on the hammer 61.

The return spring 66 is, for example, a metal spring, and is deformable (compressible) in the X-axis direction and energizes the grip portion 70 toward the proximal end side. The energizing force of the return spring 66 that is applied on the grip portion 70 is smaller than the energizing force of the push spring 62 in the initial state that is applied on grip portion 70 (a state in which the compression is not generated by the movement of the hammer 61). In this manner, when the push spring 62 is in the initial state, the grip portion 70 is positioned at the frontmost end position in the movable range (the frontmost end position in the X-axis direction in the grip portion accommodating portion 52A). Meanwhile, when the push spring 62 is compressed so that no energizing force is applied on the grip portion 70, the grip portion 70 is moved to the rearmost end position in the movable range (the rearmost end position in the X-axis direction in the grip portion accommodating portion 52A) by the energizing force of the return spring 66.

In the wire feeding device 51, the lever 81, the links 85 and 87, the joints 84, 86, and 88, the slider 89, the slider 63, the grip release drive portion 64, and the grip release portion 65 form the power transmission mechanism.

The lever 81 is a portion for a technician using the wire feeding device 51 to manually perform a turning operation. The lever 81 is turnable around the lever rotation shaft 82. The lever 81 is energized by a torsion spring 83 so that the angle formed with the housing 52 is larger. As illustrated in FIG. 21, the lever rotation shaft 82 of the lever 81 and one end of the link 85 are connected through the joint 84 such that the turning force is transmittable from the lever 81 to the link 85. In the present embodiment, the link 85 is configured to turn integrally with the turn of the lever 81.

The other end of the link 85 and one end of the link 87 are rotatably connected through the joint 86. The other end of the link 87 and the slider 89 are rotatably connected through the joint 88. The slider 63 and the grip release portion 65 are connected to the Z-axis positive direction side of the slider 89. The slider 63 and the grip release portion 65 can move linearly in the X-axis direction integrally with the slider 89.

In this power transmission mechanism, when the lever 81 is turned in an R1 direction, the link 85 is turned in an R2 direction. With the turn of the link 85, the link 87 moves with the movement of the slider 89 along the X-axis direction. In the present embodiment, the turn angle of the lever 81, the lengths of the links 85 and 87, and the like of this power transmission mechanism are adjusted such that in a turnable range of the lever 81, the slider 63 connected to the slider 89 can move in the entire moving range in the X-axis direction.

As illustrated in FIG. 19, the slider 63 has an inverted C-shaped shape on the X-Y plane and includes, at a portion on the Y-axis negative direction side, a hook 63A engageable with the protruding portion 61A of the hammer 61 and an inclined portion 63B with an inclined surface gently inclined on the Y-axis negative direction side. When the slider 63 moves to the X-axis negative direction side and comes into contact with the protruding portion 52C of the housing 52, the inclined portion 63B acts to deform the portion on the Y-axis negative direction side of the slider 63 in the Y-axis positive direction.

When the slider 63 moves from the frontmost end position to the proximal end side in the X-axis direction in the moving range of the slider 63, the hook 63A engages with the protruding portion 61A of the hammer 61. When the slider 63 further moves, the hammer 61 is moved to the proximal end side, so that the push spring 62 is compressed. When the slider 63 approaches the rearmost end position, the protruding portion 52C of the housing 52 pushes up the inclined portion 63B of the slider 63 in the Y-axis positive direction side, and the portion on the Y-axis negative direction side of the slider 63 is deformed in the Y-axis positive direction, so that the engagement between the hook 63A and the protruding portion 61A of the hammer 61 is released. As a result, the deformed state (compressed state) of the push spring 62 is released at once, and the push spring 62 pushes the hammer 61 in the X-axis direction.

The grip release portion 65 is a plate-shaped member in which the surface on the housing 52 side on the distal end side in the X-axis direction is thinner toward the distal end side. When the distal end portion of the grip release portion 65 is moved to the distal end side in the X-axis direction, the grip release portion 65 pushes a protruding portion 64A of the grip release drive portion 64 downward.

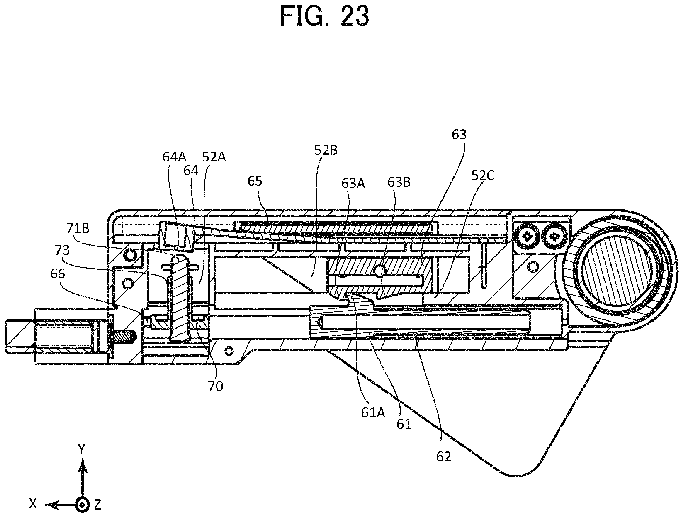

The grip release drive portion 64 is formed of, for example, an elastic member, is a rod-shaped member extending in the X-axis direction, and includes, at the distal end, the protruding portion 64A projecting to the Y-axis negative direction side. The protruding portion 64A is arranged on the Y-axis positive direction side with respect to the leg portion 71B of the grip portion 70. The distal end portion of the exclusion release drive portion 64 is energized to bend in the Y-axis positive direction side. When the distal end portion is not pushed on the Y-axis positive direction side by the grip release portion 65, the protruding portion 64A is not in contact with the leg portion 71B and does not press the leg portion 71B (see FIG. 23), while when the distal end portion is pushed on the Y-axis positive direction side by the grip release portion 65, the protruding portion 64A presses the leg portion 71B of the grip portion 70 in the Y-axis negative direction. In this case, the grip spring 73 of the grip portion 70 is compressed to move the leg portion 71B of the grip portion 70 in the Y-axis negative direction. As a result, the surface on the second component 72 side of the grip surface portion 71A of the first component 71 and the surface on the first component 71 side of the grip surface portion 72A of the second component 72 are separated from each other, thereby releasing the grip of the guide wire GW.

The following will specifically describe a use method of the wire feeding device 51 according to the second embodiment and the operation of the wire feeding device 51 when used, with reference to the drawings. In the wire feeding device 51, the grip portion 70, the hammer 61, and the slider 63 are configured to interlock with each other so that gripping the guide wire GW, moving the grip portion 70 toward the distal end direction of the guide wire GW, releasing the grip of the guide wire GW, and moving the grip portion 70 toward the rear end direction are performed in this order.

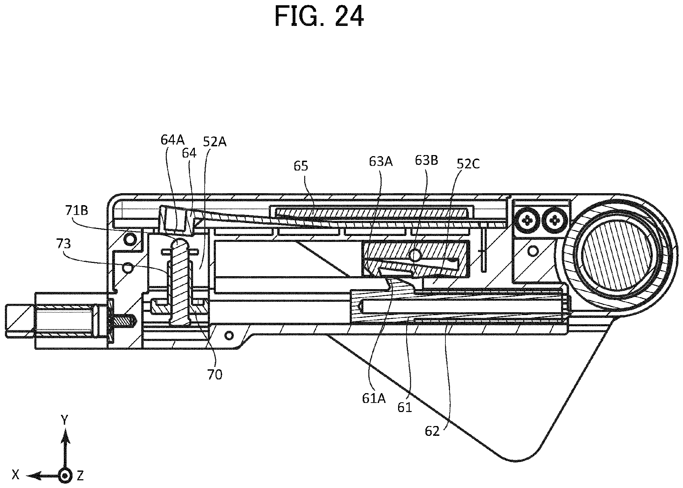

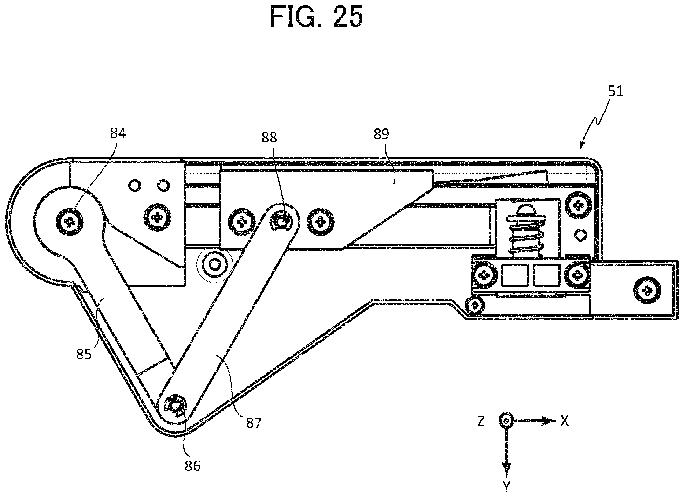

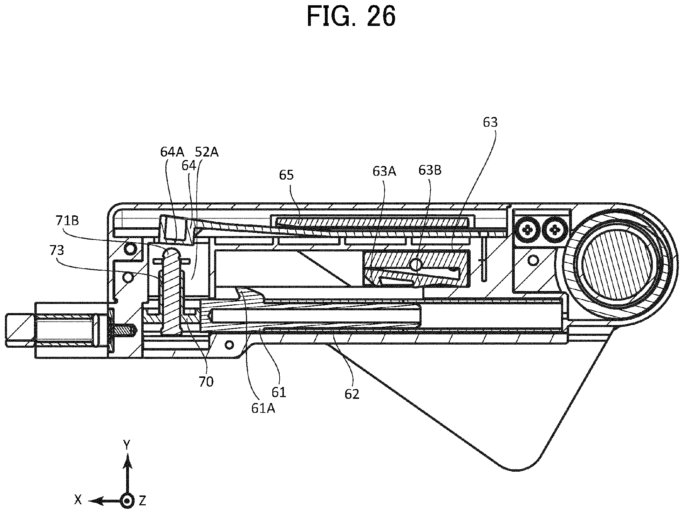

FIG. 22 is a sectional top view of the wire feeding device in a gripping preparation state. FIG. 23 is a sectional top view of the wire feeding device in a state immediately before the start of feeding. FIG. 24 is a sectional top view of the wire feeding device in a state immediately after the start of feeding. FIG. 25 is a bottom view of the wire feeding device in a state immediately after the start of feeding. FIG. 26 is a sectional top view of the wire feeding device in a state after the completion of feeding.

First, the guide wire GW is inserted into a blood vessel, and then the guide wire GW is pushed to an obstructed site along the blood vessel. Next, after the distal end of the guide wire GW reaches the obstructed site, a catheter 110 is pushed to an obstructed site using the guide wire GW as a guide. Then, the proximal end side of the guide wire GW is accommodated in the guide wire accommodating portion 54 of the housing 52 from the Z-axis positive direction side, and the connector 110 is connected to the catheter hub 102 of the catheter 110. Then, the connector 110 is pushed into the connector connection portion 53 from the Z-axis positive direction to connect the connector 110 to the wire feeding device 51.

In this case, as illustrated in FIG. 19, the grip release portion 65 pushes the grip release drive portion 64, and the grip spring 73 is compressed. Thus, the surface on the second component 72 side of the grip surface portion 71A of the first component 71 and the surface on the first component 71 side of the grip surface portion 72A of the second component 72 are separated from each other, and arrangement space is formed therebetween, and the grip of the guide wire GW by the grip portion 70 is released. Therefore, as described above, the catheter 110 with the guide wire GW inserted therethrough is attached to the wire feeding device 51, whereby the guide wire GW can be easily accommodated in the arrangement space. This can reduce the preparation time for feeding the guide wire GW by the wire feeding device 51, and reduces loads on the patient and the technician.

Next, when the lever 81 is turned slightly by gripping the lever 81 and the housing 52, the slider 63 and the grip release portion 65 slide toward the proximal end side. Because the protruding portion 61A of the hammer 61 is engaged with the hook 63A of the slider 63, as illustrated in FIG. 22, the hammer 61 is moved toward the proximal end side with the movement of the slider 63, so that the push spring 62 is compressed.

Here, the grip portion 70 is no longer pushed toward the X-axis positive direction by the hammer 61. Thus, the grip portion 70 slides to the rearmost end in the movable range by the energizing force of the return spring 66. As a result, the grip portion 70 moves by a distance D from the frontmost end (initial position) to the rearmost end in the movable range. The distance D corresponds to a feeding amount per time by the wire feeding device 51. For example, when the feeding amount per time by the wire feeding device 51 is 2 mm, the grip portion 70 slides to the rear end side by 2 mm from the initial position. Here, the grip release portion 65 is configured to be at a position where the grip spring 73 of the grip portion 70 is compressed through the grip release drive portion 64, which keeps the state in which the grip portion 70 does not grip the guide wire GW.

Further, when the lever 81 is turned by further gripping the lever 81, the slider 63 and the grip release portion 65 further slide toward the proximal end side. Because the hook 63A of the slider 63 remains engaged with the protruding portion 61A of the hammer 61, as illustrated in FIG. 23, the hammer 61 is moved toward the proximal end side with the movement of the slider 63, so that the push spring 62 is further compressed.

Here, the grip release portion 65 deviates from the position where the distal end portion of the grip release drive portion 64 is pushed. Thus, the grip release drive portion 64 is no longer in contact with the leg portion 71B and no longer compresses the grip spring 73, and the guide wire GW between the surface on the second component 72 side of the grip surface portion 71A of the first component 71 and the surface on the first component 71 side of the grip surface portion 72A of the second component 72 is gripped.

Further, as illustrated in FIG. 25, when the lever 81 is turned to the position (feeding position) where the slider 89 (the same applies to the slider 63) is at the most proximal end side of the moving range, the slider 63 and the grip release portion 65 further slide to the proximal end side. Then, as illustrated in FIG. 24, the inclined portion 63B of the slider 63 is pushed up by the protruding portion 52C of the housing 52, and the portion on the Y-axis negative direction side of the slider 63 is bent to the positive direction side. As a result, the engaging state of the hook 63A of the slider 63 with the protruding portion 61A of the hammer 61 is released.

In this manner, as illustrated in FIG. 26, the energizing force of the push spring 62 is applied at once to the movement of the hammer 61 toward the distal end direction, so that the hammer 61 moves toward the distal end direction, and the distal end side of the hammer 61 hits the proximal end side of the grip portion 70.

As a result, the grip portion 70 gripping the guide wire GW moves toward the distal end direction by the impact due to a hit with the hammer 61, and stops at the frontmost end position of the grip portion 70. Here, the grip release portion 65 is not in contact with the grip release drive portion 64 and, as a result, the grip release drive portion 64 is not in contact with the grip portion 70. Thus, the guide wire GW remains gripped.

Therefore, the grip portion 70 moves from the rearmost end position to the frontmost end position while keeping the state of gripping the guide wire GW. As a result, the guide wire GW is fed to the distal end side by the distance D from the rearmost end position to the frontmost end position of the grip portion 70.

Thereafter, when the technician stops gripping the lever 81, the lever 81 is turned in the opposite direction by the torsion spring 83 to restore the initial state illustrated in FIG. 21. With this, the slider 63 and the grip release portion 65 slide toward the distal end side to restore the initial state illustrated in FIG. 19. Note that if it is necessary to further feed the guide wire GW, the lever 81 may be further turned by gripping the lever 81.

As described above, in the wire feeding device 51 according to the present embodiment, it is possible to feed the guide wire GW by only an appropriate amount by applying impact force due to the energizing force accumulated in the push spring 62 on the guide wire GW. In this manner, it is possible to apply impact force on the guide wire GW, which allows the guide wire GW to penetrate an occluding object more effectively.

The following will describe an operation of the wire feeding device 51 when the lever 81 is gripped so that the grip portion 70 grips the guide wire GW, as illustrated in FIG. 23, and then the feeding of the guide wire GW is cancelled.

In the state illustrated in FIG. 23, when gripping the lever 81 is stopped, the torsion spring 83 causes the lever 81 to turn in the opposite direction. In this case, with the opposite direction of the lever 81, the slider 89, the slider 63, and the grip release portion 65 slide toward the distal end side. Here, the slider 89, the slider 63, and the grip release portion 65 slide toward the distal end side while keeping the engaging state of the hook 63A of the slider 63 with the protruding portion 61A of the hammer 61.

In this case, as illustrated in FIG. 22, the grip release portion 65 pushes the protruding portion 64A of the grip release drive portion 64 in the Y-axis negative direction before the hammer 61 hits the proximal end side of the grip portion 70. As a result, the grip spring 73 is compressed, so that the grip portion 70 does not grip the guide wire GW. Therefore, when the hammer 61 comes into contact with the proximal end side of the grip portion 70 and moves the grip portion 70 to the position illustrated in FIG. 19, the guide wire GW is not fed to the distal end side.

As described above, in the wire feeding device 51 according to the present embodiment, even when the lever 81 is gripped so that the guide wire GW is gripped once, if the lever 81 is not moved to the guide wire GW feeding position, the initial state can be restored without feeding the guide wire GW to the distal end side by stopping gripping the lever 81. Therefore, even after the lever 81 is gripped once to some extent, the grip of the guide wire GW can be released, thereby allowing the change or the like of the directions of the catheter 110 and the guide wire GW. In this manner, it is possible to perform an appropriate procedure in accordance with situations, and reduce loads on the patient and the technician.

The technology disclosed in this specification is not limited to the above-described embodiments, and can be modified in various forms without departing from the gist thereof. For example, the following modifications are also possible.

In the above-described embodiments, metal springs are used as the push springs 16 and 62. However, other kinds of elastic bodies such as rubber cords and plate springs may be used. The material of the elastic body may be a resin material. In the above-described embodiment, the hammers 15 and 61 are also made of metal. However, they may be made of a resin material, similarly to the push springs.

In the above-described embodiments, the hammers 15 and 61 are moved to compress the push springs 16 and 62, thereby increasing the energizing force on the hammers 15 and 61 toward the distal end direction. However, an elastic body may be provided to extend with the movement of the hammers 15, 61 toward the proximal end side, so that the energizing force is increased by the extension of the elastic body, for example.

In the above-described embodiments, it is possible to provide a mechanism for adjusting the movable range of the grip portions 10 and 70 in the X-axis direction, such as a mechanism for moving the position in the X-axis direction of the wall that determines the movable range, for example. In this manner, it is possible to easily and appropriately adjust the wire feeding amount by the wire feeding device.

In the above-described embodiment, it is possible to provide a mechanism for adjusting the compression amount of the push springs 16 and 62 in the initial state, such as a mechanism for moving the position of the wall on the proximal end side of the push springs 16 and 62, for example. In this manner, it is possible to easily and appropriately adjust the impact force applied on the grip portions 10 and 70 by the hammers 15 and 61 of the wire feeding device.

In the above-described embodiment, the technician manually rotates (turns) the handle 31 (or lever 81) to feed the guide wire GW. However, a power-operated motor may be used to feed the guide wire GW. For example, the link 32 (or the link 85) may be rotated (turned) by the power of a motor. In this case, the motor may be stopped when the link 32 is rotated once, or the motor may be stopped when the link 85 is turned by a predetermined angle. For example, a switch for driving the motor may be provided, so that when this switch is pressed once, the motor may be driven to rotate the link 32 once or turn it by a predetermined angle.

Note that the present inventions are not limited to the configurations of the above-described embodiments, but is defined by the terms of the claims and is intended to include any modifications within the scope and meaning equivalent to the terms of the claims.

### REFERENCE SIGNS LIST

1, 51 wire feeding device
5, 52 housing
5A through hole
5B, 52A grip portion accommodating portion
5C, 52B slider accommodating portion
5D, 52C protruding portion
6 adapter
10, 41, 44, 47, 70 grip portion
11, 42, 45, 48, 71 first component
11A, 42A, 45A, 48A protruding portion
11B, 71B leg portion
12, 43, 46, 49, 72 second component
12A concave portion
12B protruding portion
12C, 72C through hole portion
13, 73 grip spring
15, 61 hammer
15A, 61A protruding portion
16, 62 push spring
17, 66 return spring
20, 63, 89 slider
21, 83 torsion spring
22, 63A hook
23, 65 grip release portion
24 holding portion
31 handle
32, 33, 85, 87 link
34, 35, 36, 84, 88 joint
52D partial housing
53 connector connection portion
53A, 53B connection piece
54 guide wire accommodating portion
63B inclined portion
64 grip release drive portion
64A protruding portion
72A, 72B grip surface portion
72B concave portion
81 lever
82 lever rotation shaft
101 catheter
102 catheter hub
102A rear end portion
110 connector
110A dial portion
110B through hole
110C attachment portion
110D rear end portion
GW guide wire

## Claims

1. . A wire feeding device feeding a wire in a distal end direction, the wire feeding device comprising:
a grip portion that is able to grip the wire and cancel a grip of the wire, and is movable in the distal end direction and a proximal end direction;
a hitting portion that is arranged on a proximal end side of the grip portion, and is movable in an axial direction of the wire and is able to come into contact with and separate from the grip portion;
an elastic body that is able to energize the hitting portion toward the distal end direction;
an energizing portion that deforms the elastic body and increases energizing force on the hitting portion toward the distal end direction; and
a release portion that releases a deformed state of the elastic body with the energizing force increased by the energizing portion, wherein
the wire gripped by the grip portion is fed toward the distal end direction by causing the hitting portion to hit the grip portion by the energizing force of the elastic body whose deformed state is released by the release portion and moving the grip portion toward the distal end direction.

2. The wire feeding device according to claim 1,
wherein the grip portion, the hitting portion, and the release portion are configured to interlock with each other so that gripping the wire, moving the grip portion toward the distal end direction by a hit of the hitting portion with the grip portion, releasing the grip of the wire, and moving the grip portion toward the proximal end direction are performed in this order.

3. The wire feeding device according to claim 1 or 2,
wherein the grip portion is movable in a given movable range in the axial direction, and is configured to keep, when moved to a frontmost end position in the distal end direction in the movable range by a hit of the hitting portion, gripping the wire at the position.

4. The wire feeding device according to claim 3, further comprising:
a release and moving portion that cancels the grip of the wire by the grip portion at the frontmost end position, and moves the grip portion from the frontmost end position to a rearmost end position in the movable range while keeping a cancelled state of the grip of the wire by the grip portion.

5. The wire feeding device according to claim 4, further comprising:
a power transmission mechanism that enables, with power supplied from a same power source, an operation of moving the grip portion from the frontmost end position to the rearmost end position by the release and moving portion and an operation of increasing energizing force of the elastic body by the energizing portion, as a sequence of operations.

6. The wire feeding device according to any one of claims 1 to 5, wherein
the elastic body is able to increase energizing force with the movement of the hitting portion toward the proximal end side,
the energizing portion includes a hook engageable with a protruding portion of the hitting portion, and is able to move the hitting portion toward the proximal end side while the hook is engaged with the protruding portion, and
the release portion releases the deformed state of the elastic body by releasing the engagement between the hook of the energizing portion and the protruding portion of the hitting portion.

7. The wire feeding device according to any one of claims 1 to 6,
wherein the grip portion includes facing grip surfaces that are capable of gripping the wire by sandwiching the wire, and
the grip surface includes a surface intersecting the movable direction of the grip portion.

8. The wire feeding device according to any one of claims 1 to 7, wherein the grip portion has arrangement space for arranging the wire to be gripped, and
the arrangement space is openable to an outside through an opening extending over the entire movable direction of the grip portion.

9. The wire feeding device according to any one of claims 1 to 7,
wherein the grip portion has arrangement space for arranging the wire to be gripped, and
the arrangement space is able to form a through hole extending in the movable direction of the grip portion.

10. The wire feeding device according to claim 2, further comprising:
a grip release portion at the time of stopping that cancels, when energization of the energizing force by the energizing portion is stopped, the grip of the wire by the grip portion while the grip portion does not move to the distal end direction.

11. The wire feeding device according to claim 5,
wherein the power source is a power-operated motor.

12. The wire feeding device according to claim 5, further comprising:
a lever that is able to supply power to the power transmission mechanism by turning around a given axis in accordance with technician's operation.
